# EUROPEAN PATENT APPLICATION

(11) **EP 1 772 146 A1**
(43) Date of publication of application: **11.04.2007**
(21) Application number: 05380212.0
(22) Date of filing: 03.10.2005
(51) Int. Cl.: A61K 31/202, A61P 25/00, A61P 25/28

(54) **Polyunsaturated fatty acids as bace inhibitors**

(71) Applicant: Neuropharma, S.A., 28760 Madrid (ES)
(72) Inventor: Martinez Gil, Ana, 28004 Madrid (ES); Castro Morera, Ana, 28028 Madrid (ES); Alonso Gordillo, Diana, 28230 Las Rozas, Madrid (ES); Exposito Castro, Angeles, 28794 Guadalix de la Sierra - Madrid (ES); Gomez Gomez, Cristina, 28700 San Sebastian de los Reyes,Madrid (ES); Medina, PAdilla, Miguel, 28029 Madrid (ES); Garcia Palomero, Esther, 28911 Leganes - Madrid (ES); Valenzuela Liminana, Rita, 28027 Madrid (ES)
(74) Representative: ABG Patentes, S.L.

(57) **Abstract**

Use of polyunsaturated fatty acids of formula (I) for preventing or treating BACE mediated diseases or conditions, specially neurodegenerative disease such as Alzheimer's Disease, and pharmaceutical compositions comprising polyunsaturated fatty acids of formula (I).

## Description

### FIELD OF THE INVENTION

The present invention relates to polyunsaturated fatty acids as enzyme inhibitors, and more particularly to inhibitors of β site AβPP cleaving enzyme, BACE, and to their use for the treatment and or prevention of a disease in which BACE is involved, such as neurodegenerative diseases, specially Alzheimer's Disease, and to pharmaceutical compositions comprising said fatty acids.

### BACKGROUND OF THE INVENTION

### FATTY ACIDS

Fatty acids consist of the elements carbon (C), hydrogen (H) and oxygen (O) arranged as a carbon chain skeleton with a carboxyl group (-COOH) at one end. Saturated fatty acids (SFAs) have all the hydrogen that the carbon atoms can hold, and therefore, have no double bonds between the carbons. Monounsaturated fatty acids (MUFAs) have only one double bond. Polyunsaturated fatty acids (PUFAs) have more than one double bond.

The numbers at the beginning of the scientific names indicate the locations of the double bonds. By convention, the carbon of the carboxyl group is carbon number one. Greek numeric prefixes such as di, tri, tetra, penta, hexa, etc., are used as multipliers and to describe the length of carbon chains containing more than four atoms.

The double bonds give rise to configurational isomers cis and trans. Omega-3 (ω3) and omega-6 (ω6) fatty acids are unsaturated "Essential Fatty Acids" (EFAs) that need to be included in the diet because the human metabolism cannot create them from other fatty acids. Since these fatty acids are polyunsaturated, the terms n-3 PUFAs and n-6 PUFAs are applied to omega-3 and omega-6 fatty acids, respectively. These fatty acids use the Greek alphabet (α,β,γ,...,ω) to identify the location of the double bonds. The "alpha" carbon is the carbon closest to the carboxyl group (carbon number 2), and the "omega" is the last carbon of the chain.

For example, linoleic acid is an omega-6 fatty acid; it plays an important role in lowering cholesterol levels. Alpha-linolenic acid is an omega-3 fatty acid.

At room temperature (25°C), most unsaturated fatty acids are oily liquids.

DHA (docosahexaenoic acid) and AA (arachidonic acid) both crucial to the optimal development of the brain and eyes. In fact, DHA and AA are routinely added to infant formulas, due to their importance in infant nutrition. Particularly, DHA is known to be required for memory formation, excitable membrane function, photoreceptor cell biogenesis and function, and neuronal signaling (Brain Pathol., "Neuroprotectin D1 (NP01): a DHA-derived mediator that protects brain and retina against cell injury-induced oxidative stress", 15(2), 159-166). In fact, DHA makes up a large proportion of the brain's lipids, and is the predominant omega-3 fatty acid found in this organ.

Many studies have shown that these PUFAs are crucial components of the diet; for example DHA is an omega-3 polyunsaturated fatty acid found predominantly in coldwater fish (for example mackerel, sardines and salmon) and algae. In general, these PUFAs have to be provided by the diet, as it has a low conversion rate in mammals from dietary omega-3 sources. Excessive amounts of omega-6 polyunsaturated fatty acids and a very high omega-6/omega-3 ratio have been linked with pathogenesis of many diseases, including cardiovascular disease, cancer, and inflammatory and autoimmune diseases. The ratio of omega-6 to omega-3 in modern diets is approximately 15:1, whereas ratios of 2:1 to 4:1 have been associated with reduced mortality from cardiovascular disease, suppressed inflammation in patients with rheumatoid arthritis, and decreased risk of breast cancer.

There has also been established a relation between blood levels of omega-3 PUFAs and neuropsychiatric disorders (Reprod. Nutr. Dev., "Omega-3 fatty acids and neuropsychiatric disorders", 45, 1-28), for example Schizophrenia, Depression, Mood Disorders, Attention Deficit Disorder, Hyperactivity, Dementia and Alzheimer's Disease. Nevertheless, it has not yet been completely elucidated which is the exact relationship: it could be related to an insufficient dietary supply, or to an abnormally elevated lipid oxidation. Some studies have also stated that an omega-3 PUFA rich diet may have neuroprotective effects and even prevent or postpone some neurodegenerative diseases; for example, diets comprising fish or fish oil rich in omega-3 PUFAs may have this protective effect. In this sense, some studies with Animal Models have been performed (Lim et al., "A diet enriched with the omega-3 fatty acid docosahexaenoic acid reduces Amyloid burden in an aged Alzheimer mouse model", The Journal of Neuroscience, 25(13), 2005); also some studies regarding omega-3 PUFA rich diet in human and incidence of some neurodegenerative diseases have been established (Morris et al., "Consumption of fish and n-3 fatty acids and risk of incident Alzheimer Disease", Arch. Neurol., 60(7), 940-946; Horrocks et al, "Health benefits of docosahexaenoic acid (DHA)", Pharmacol. Res., 40(3), 211-225, 1999). All the studies have been performed based on omega-3 PUFAs.

### BETA AMYLOID

Alzheimer's Disease (AD) is a neurodegenerative disorder characterized by the presence of β-Amyloid protein deposits in the core of neuritic plaques and abnormal neurofibrillary tangles in the brain of AD patients. The Amyloid β-protein (Aβ) is formed by two endoproteolytic cleavages of the Amyloid β protein precursor (AβPP), a large transmembrane type I protein. A protease termed β-secretase cleaves AβPP at the N-terminus of the Aβ domain to generate the soluble AβPP and the membrane anchored C-terminal fragments (CTFs). Then, a second secretase called γ-secretase, cuts CTFs within the transmembrane region to form Aβ, to form Aβ, which is secreted from the cells. The identification of compounds able to prevent or reduce this event has become an important goal for the research on the treatment of AD.

Also other diseases have been linked to the presence of beta Amyloid deposits in the brain. Some examples are MCI (mild cognitive impairment), Down's syndrome, Hereditary Cerebral Hemmorhage with Amyloidosis of the Dutch-Type, cerebral Amyloid angiopathy, other degenerative dementias, including dementias of mixed vascular and degenerative origin, dementia associated with Parkinson's disease, dementia associated with progressive supranuclear palsy, dementia associated with cortical basal degeneration, and diffuse Lewy body type Alzheimer's disease (see publication US20040132782).

### BACE

BACE (β site AβPP cleaving enzyme) is an aspartyl protease with β-secretase activity. BACE is a type I integral membrane protein with a typical aspartyl protease motif in its luminal domain. BACE hydrolyzes AβPP specifically at the Met-Asp site, with an acidic pH optimum. BACE is highly expressed in the brain and it colocalizes with the intracellular sites of CTFs and Aβ production. BACE has become an important target for the development of therapeutic compounds against Alzheimer's Disease.

There are several factors that increase the expression and activity of BACE. Oxidant agents and oxidative products, such as H₂O₂ or HNE (4-hidroxynonenal), which is an aldehydic end product of polyinsaturated fatty acids, were shown to increase intracellular and secreted Aβ levels in neuronal and non neuronal cells (Paola *et al.* 2000; Misonou *et al.* 2001; Frederikse *et al.* 1996). Many studies have been carried out to determine the cellular mechanisms that underlie the Aβ overproduction. In 2002, Tamagno et al.(Oxidative Stress Increases Expression and Activity of BACE in NT2 Neurons, 2002, Neurobiol. Dis., 10, 279-288) demonstrated that oxidative stress induces BACE protein levels and activity, and this event is mediated by the oxidative product HNE. According to this study, exposure of NT₂ cells to oxidant agents did not influence AβPP expression. The effect of these agents on Aβ is related to an increase of BACE1 expression via transcriptional up regulation of BACE1 gene *(*Oxidative stress potentiates BACE1 gene expression and Aβ generation, Tong et al., 2004, J. Neural. Transm., 112(3):455-69).

The identification of compounds which are able to prevent the effect of oxidative agents has become an important goal of current research in Alzheimer's Disease. Among these compounds, dehydroepiandrosterone (DHEA) and its role in the CNS have been studied by Tamagno et al. *(*Dehydroepiandrosterone reduces expression and activity of BACE in NT2 neurons exposed to oxidative stress, Tamagno et al., 2003, Neurobiol. Dis., 14, 291-301). DHEA is an adrenal steroid that serves as a precursor to both androgens and estrogens and is synthesized from sterol precursors in the nervous system (Balieu 1981). DHEA is known to improve a variety of functional activities in the CNS, including increased memory and learning in different animal models (Vallée et al. 2001) and exerts protection against excitatory amino acids and Aβ neurotoxicity. In this study, it has been demonstrated that a pre-treatment with DHEA is able to decrease the expression, protein levels and activity of BACE induced in NT₂ neurons by oxidative agents, such as Asc/Fe and H₂O₂/Fe. This protection seems to be due to the antioxidant properties of the steroid, able to prevent the production of the end products of lipid oxidation, such as HNE. The oxidative stress products induce an increase of BACE protein levels and activity, and this induction is due to a gene overexpression, as has been demonstrated by quantitative PCR analysis. Decline of DHEA concentrations with ageing led to the suggestion that it could be implicated in longevity and that its progressive decrease can be related with some of the aging-related degenerative disorders, including AD. In conclusion, DHEA is able to prevent the oxidative stress-dependent Amyloidogenic processing of AβPP through the negative modulation of the expression and activity of BACE.

The expression of BACE has been localized in the brain, in particular in neurons, indicating that neurons are the major source of β-Amyloid peptides in the brain. Astrocytes, on the other hand, are known to be important for β-Amyloid clearance and degradation, for providing trophic support to neurons and for forming a protective barrier between β-Amyloid deposits and neurons. However, according to Rossner et al. *(*Alzheimer's disease β-secretase BACE1 is not a neuron specific enzyme, Rossner et al., J Neurobiochem. 2005, 92, 226-234), astrocytes may also represent an alternative cellular source of β-Amyloid peptides. The role of astrocytes in the pathogenesis of AD remains undetermined and may differ on a case to case instance due to dependence on a broad spectrum of interactive events in neurons, astrocytes and microglia.

### SUMMARY OF THE INVENTION

It has now surprisingly been found that polyunsaturated fatty acids show an inhibition upon BACE; particularly, compounds of formula (I) 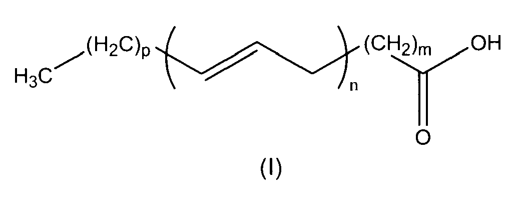 wherein
m=1-10
n=2-6
p=1-7
have been found to inhibit BACE activity at concentrations as low as 10⁻⁵ and 10⁻⁶ M.

Therefore, in a first aspect, the present invention is related to the use of the compounds of formula (I) in the preparation of a medicament for a BACE mediated disease or condition.

As the compounds of formula (I) exhibit activity as inhibitors of BACE, they have the ability to halt or reduce the production of Amyloid beta and thus reduce or eliminate the formation of Amyloid beta deposits in the brain. Thus, the compounds may be useful for treating humans or animals suffering from a condition characterized by a pathological form of Amyloid beta peptide, such as Amyloid beta plaques, and for helping to prevent or delay the onset of said condition. Thus, the compounds of formula (I) according to the present invention may be useful for preventing, delaying and treating patients with Alzheimer's Disease, MCI (mild cognitive impairment), Down's syndrome, Hereditary Cerebral Hemmorhage with Amyloidosis of the Dutch-Type, cerebral Amyloid angiopathy. They may also be useful for treating other degenerative dementias, including dementias of mixed vascular and degenerative origin, dementia associated with Parkinson's disease, dementia associated with progressive supranuclear palsy, dementia associated with cortical basal degeneration and diffuse Lewy body type Alzheimer's disease.

In a second aspect, the present invention is related to a pharmaceutical composition comprising a therapeutically effective amount of one or more compounds of formula (I) as defined above.

According to an additional aspect, the invention is related to a method for inhibiting BACE activity in a biological sample, comprising contacting said biological sample with an effective inhibitory amount of a compound of formula (I) as defined above.

A last aspect of the present invention is a method for treating or preventing a BACE mediated disease or condition comprising administering to a patient a therapeutically effective amount of one or more compounds of formula (I) as defined above.

### DETAILED DESCRIPTION OF THE INVENTION

The oceans are the source of a large group of structurally unique natural products that are mainly accumulated in invertebrates such as sponges, tunicates, bryozoans, and mollusc [Burkhard Haefner, Drug Dis. Today. 2003,8,2,536-544]. Several of these compounds show pronounced pharmacological activities and are interesting candidates for new drugs in several areas of treatment [DJ. Newman, GM.Cragg, KM.Snader, J.Nat.Prod. 2003, 66, 1022-1037].

Within the aim of isolating compounds from marine organisms which show biological activities which are interesting for preventing and/or treating neurodegenerative diseases, it was found that isopropanolic extracts from a number of marine organisms, especially seastars (for example, *perknaster aurorae,* but also of other species, for example *cardisoma* (particularly the digestive apparatus of these blue crabs) presented interesting biological activities, particularly inhibiting BACE. Further fractioning and structure elucidation led to find out that some of the compounds which were responsible of the activity in the initial isopropanolic extracts were polyunsaturated fatty acids falling within the general formula (I) of the present invention. Particularly, the monodimensional and bidimensional proton and carbon nuclear magnetic resonance studies and mass spectra performed to elucidate the structure of the compounds which were isolated more frequently, were correspondent with those of known PUFAs, namely linolenic acid, linoleic acid, eicosapentaenoic acid and arachidonic acid. Taking advantage of the fact that these fatty acids are commercially available, the positive results of the biological activity obtained from the natural extracts were checked by testing some commercially available PUFAs. In fact their activity was confirmed by the additional testing.

Therefore, as indicated above, in a first aspect, the present invention is related to the use of a compound of formula (I) 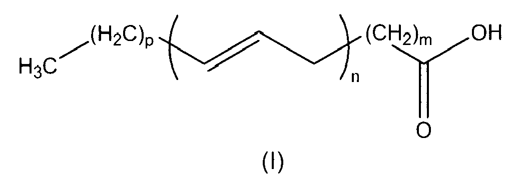 wherein
m=1-10
n = 2-6
p=1-7
in the manufacture of a medicament for the treatment and/or prevention of a BACE mediated disease or condition.

The compounds of formula (I) may include isomers depending on the presence of the double bonds (e.g. cis, trans). The single isomers and mixtures thereof fall within the scope of the present invention. Unless otherwise stated, the compounds of formula (I), in all the aspects of the invention, are also meant to include compounds which differ only in the presence of one or more isotopically enriched atoms. For example, compounds having the present structures except for the replacement of a hydrogen by a deuterium or tritium, or the replacement of a carbon by a 13C- or 14C-enriched carbon are within the scope of this invention.

According to a preferred embodiment, p is from 2 to 7.

Preferably, p is either 4 or 7: the resulting fatty acid thus is, respectively, an omega-6 or an omega-9 polyinsaturated fatty acid.

In another preferred embodiment, p is 1, the compound thus being an omega-3 PUFA.

According to a preferred embodiment, the total number of carbon atoms in the compound of formula (I) is an even number. Most naturally occurring polyinsaturated fatty acids have an even carbon atoms number.

According to another preferred embodiment, m in formula (I) m is an even number, thus being selected from 2, 4 and 6.

Preferably, the compound of formula (I) is one of the following compounds:

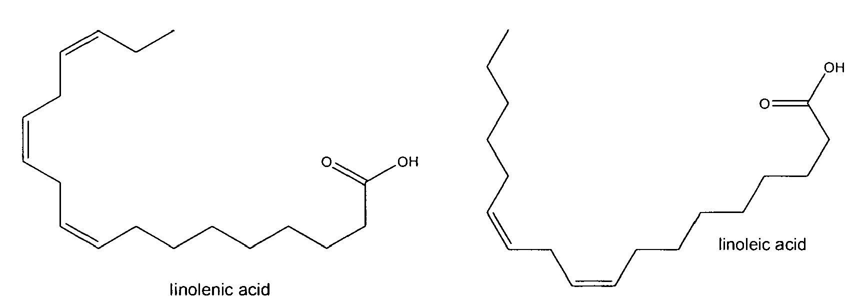

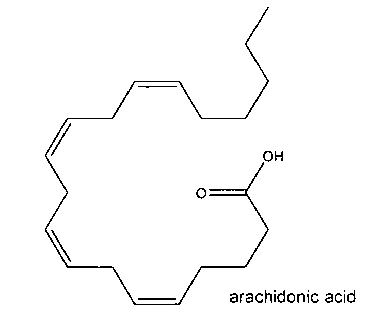

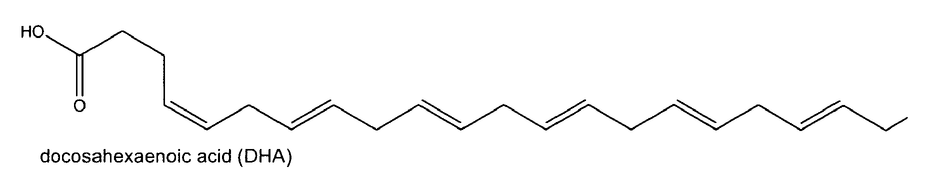

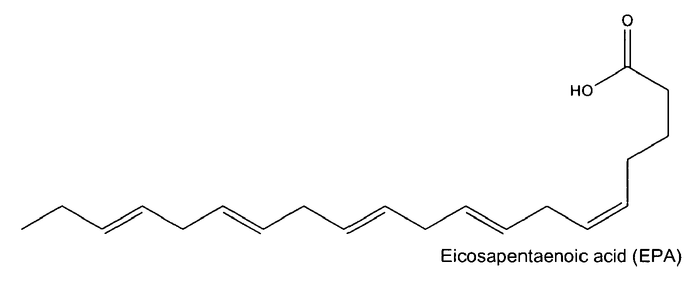

Even more preferably, the compound of formula (I) is arachidonic acid.

The IUPAC nomenclature of the above detailed fatty acids is the following:

| | |
|---|---|
| Linolenic acid | *cis,cis,cis-*9,12,15-Octadecatrienoic acid |
| Linoleic acid | *cis*-9,*cis*-12-Octadecadienoic acid |
| Arachidonic acid | cis,cis,cis,cis-5,8,11,14-Eicosatetraenoic acid |
| Docosahexaenoic acid (DHA) | cis-4,7,10,13,16,19-Docosahexaenoic Acid |
| Eicosapentaenoic acid (EPA) | cis-5, 8, 11, 14, 17-eicosapentaenoic acid |

The BACE mediated disease or condition preferably involves overproduction of Amyloid β in neuronal and non neuronal cells. In two preferred embodiments, said cells are neurons and the disease or condition involves the formation of Amyloid beta deposits in the brain.

Taking into account that the compounds of formula (I) as defined in the present invention inhibit BACE activity, they have the ability to halt or reduce the production of Amyloid beta and thus to reduce or eliminate the formation of Amyloid beta deposits in the brain. Thus, the compounds may be useful for treating humans or animals suffering from a condition characterized by a pathological form of Amyloid beta peptide, specially the formation of Amyloid beta deposits in the brain, such as Amyloid beta plaques, and for helping to prevent or delay the onset of said condition. Thus, the compounds of formula (I) according to the present invention may be useful for preventing, delaying and treating patients with Alzheimer's Disease, MCI (mild cognitive impairment), Down's syndrome, Hereditary Cerebral Hemmorhage with Amyloidosis of the Dutch-Type, cerebral Amyloid Angiopathy. They may also be useful for treating other degenerative dementias, including dementias of mixed vascular and degenerative origin, dementia associated with Parkinson's disease, dementia associated with progressive supranuclear palsy, dementia associated with cortical basal degeneration and diffuse Lewy body type Alzheimer's disease.

Preferably, the disease or condition is a neurodegenerative disease, specially Alzheimer's Disease.

One further aspect of the invention is a pharmaceutical composition comprising one or more of the compounds of formula (I). This pharmaceutical composition may be used for preventing and/or treating any disease or condition as detailed within the present invention. As indicated, a compound of formula (I) may be incorporated into the composition either alone or in admixture with one or more of them.

The PUFAs may be preferably administered either in an oral or parenteral pharmaceutical composition. Suitable dosage forms include, but are not limited to, hard and soft gelatin capsules, emulsions and microemulsions and non aqueous solutions. Oral administration is preferred because of the convenience for the patient and the chronic character of many of the diseases to be treated.

Taking into account that most PUFAs are unstable oils at room temperature, due care has to be taken when preparing the compositions in order to avoid PUFA degradation. Therefore, the pharmaceutical compositions shall be prepared by using suitable vehicles, known in the State of the Art. The compositions may include, but are not limited to, the following components:
- Diluents / carriers, such as pharmaceutical grade vegetable and/or fish oils, glicerol, solids with large specific surface area, different grades of polyethylen glycols, ethanol, water etc.
- Antioxidants, such as butylated hydroxyanisol (BHA), butylated hydroxytoluene (BHT), propyl gallate, vitamin E (alpha tocopherol), etc.
- Surfactants, such us polyoxyethylene castor oil derivatives, polysorbates, sorbitan esters, etc.
- Other excipients (e.g.flavouring)

Generally, an effective administered amount of a compound of formula (I) or an admixture of two or more of them, will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 10 mg/day to 10 gr/day.

The compounds and compositions of this invention may be used together with other drugs to provide a combination therapy. The other drugs may form part of the same composition, or be provided as a separate composition for administration at the same time or at different time.

According to a further aspect, the present invention is related to a method for inhibiting BACE activity in a biological sample, comprising contacting said biological sample with an effective inhibitory amount of one or more compounds of formula (I) as defined above.

Another aspect of the invention is a method for treating or preventing a BACE mediated disease or condition which comprises administering to a patient a therapeutically effective amount of one or more compound of formula (I) as defined above.

Preferably, the BACE mediated disease or condition is selected from Alzheimer's Disease, mild cognitive impairment, Down's syndrome, Hereditary Cerebral Hemmorhage with Amyloidosis of the Dutch-Type, cerebral Amyloid angiopathy, degenerative dementias, including dementias of mixed vascular and degenerative origin, dementia associated with Parkinson's disease, dementia associated with progressive supranuclear palsy, dementia associated with cortical basal degeneration and diffuse Lewy body type Alzheimer's disease.

In the following, the present invention is further illustrated by examples. They should in no case be interpreted as a limitation of the scope of the invention as defined in the claims.

### EXAMPLES

### BACE ASSAY PROTOCOL

The aim of this assay is to determine if a compound, either synthetic or of marine origin, is a BACE-1 inhibitor, to avoid the formation of Aβ. This assay is based on FRET technology (Fluorescence Resonance Energy Transfer). FRET is used to measure cleavage of a peptide substrate, among other uses. The peptide substrate shows two fluorophores, a fluorescence donor and a quenching acceptor. The distance between these two fluorophores has been selected so that upon light excitation, the donor fluorescence energy is significantly quenched by the acceptor. When a substrate peptide cleavage occurs, the energy balance is broken and all the donor fluorescence can be observed. The increase in fluorescence is linearly related to the rate of proteolysis (Gordon, GW et al., 1998). In this assay the reaction occurs between an enzyme, purified BACE-1, and a fluorogenic peptidic substrate who present the "Swedish mutation". The peptide cleavage by BACE-1 produces fluorescence energy and enzymatic activity can be quantified.
The reagents which are used in this assay are the following:
- rhBACE-1 β-Secretase recombinant human (R&D Systems. Ref. 931-AS).
- Fluorogenic Peptide Substrate IV (R&D Systems. Ref. ES004).
- B-SECRETASE INHIBITOR H-4848. (BACHEM. Ref. H-4848.0001).
- FLUOSTAR OPTIMA plate reader with 320 nm excitation filter and 405 nm emission filter (Biogen cientifica).
- Black microwell plate, 96 wells (Nunc. Ref. 00006877). Supplier: Dismadel.
- Sodium acetate.

The assay is carried out in a 96 wells microplate. The final concentration of substrate is 3,5 µM per well, and the enzyme concentration is 0,5 µg/ml. The final volume of the assay is 100 µl per well and all reagents are diluted in Reaction Buffer. The compounds are tested at a concentration of 10⁻⁵. The control in the assay is the commercial inhibitor β-Secretase inhibitor H-4848, which is tested at 300 nM. All the samples and controls are studied by duplicate.

The plate is mixed gently and changes in the fluorescence are measured using the plate reader, with "BACE" protocol. The temperature should be preferably maintained between 25 and 30 °C. Measurements are carried out every ten minutes during an hour. The first measure is subtracted from the last to calculate the fluorescence increase, evaluating the enzymatic activity. The 100% activity is calculated as the mean of the results of wells without sample or inhibitor.

Further information regarding this assay may be found in the following references, which are incorporated by reference into the present application:
Andrau, D et al; "BACE1- and BACE2-expressing human cells: characterization of beta-Amyloid precursor protein-derived catabolites, design of a novel fluorimetric assay, and identification of new in vitro inhibitors". J Biol Chem. 2003 Jul 11;278(28):25859-66.
   Gordon, GW et al; "Quantitative fluorescence resonance energy transfer measurements using fluorescence microscopy." Biophys J. 1998 May; 74:2702-13.

### Examples 1-4

In order to determine their activity as BACE inhibitors, a series of fatty acids of formula (I) were subjected to the above detailed protocol. The assays were performed at two different concentrations, namely 10⁻⁵M and 10⁻⁶M. The mean results of percentage of BACE Activity are shown in Table 1:

**Table 1**

| **Common Name** | **IUPAC Nomenclature** | **% BACE Activity (10⁻⁵ M)** |
|---|---|---|
| Linolenic acid | cis,cis,cis-9,12,15-Octadecatrienoic acid | 55.5 |
| Linoleic acid | *cis*-9,*cis*-12-Octadecadienoic acid | 55 |
| Arachidonic acid | cis,cis,cis,cis-5,8,11,14-Eicosatetraenoic acid | 48.7 |
| Docosahexaenoic acid (DHA) | cis-4,7,10,13,16,19-Docosahexaenoic Acid | 82 |

### Comparative examples 5-9

In order to further show the activity of the fatty acids according to formula (I), some close derivatives of arachidonic acid were also tested as comparative examples. As shown in the following, slight modifications on the acid group of the compounds of formula (I) annul their activity as BACE inhibitors. The mean results are shown in Table 2:

**Table 2**

| **Common Name** | **IUPAC Nomenclature** | **% BACE Activity (10⁻⁵ M)** |
|---|---|---|
| Anandamide | Icosa-5,8,11,14-tetraenoic acid (2-hydroxy-ethyl)-amide | 100 |
| Arachidonylcyclopropylamide (ACPA) | Cyclopropanecarboxylic acid icosa-5,8,11,14-tetraenylam ide | 100 |
| Arachidonyl alcohol | Icosa-5,8,11,14-tetraen-1-ol | 100 |
| Arachidonyl Acetate | Acetic acid icosa-5,8,11,14-tetraenyl ester | 100 |
| Ethyl arachidonate | 5,8,11,14-Eicosatetraenoic acid ethyl ester | 100 |

## Claims

1. Use of a compound of formula (I) 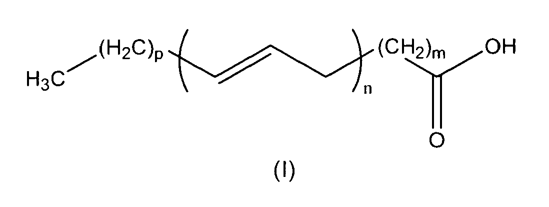 wherein
m=1-10
n=2-6
p = 1-7
in the manufacture of a medicament for the treatment and/or prevention of a BACE mediated disease or condition.

2. Use according to claim 1, wherein p=2-7.

3. Use according to any one of claims 1 or 2, wherein p=4.

4. Use according to any one of claims 1 or 2, wherein p=7.

5. Use according to claim 1, wherein p=1.

6. Use according to any one of claims 1 to 5, wherein the total number of carbon atoms in the compound of formula (I) is an even number.

7. Use according to any one of claims 1 to 6, wherein in the compound of formula (I) n is any of 2 to 5.

8. Use according to any one of claims 1 to 7, wherein in the compound (I) m is selected from any of 2, 4 or 6.

9. Use according to claim 1, wherein the compound of formula (I) is selected from the group consisting of:

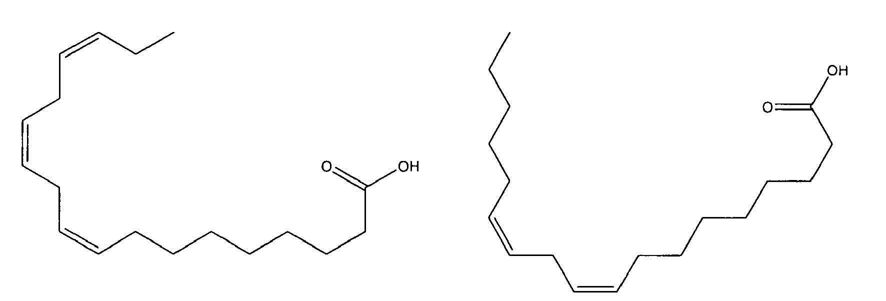

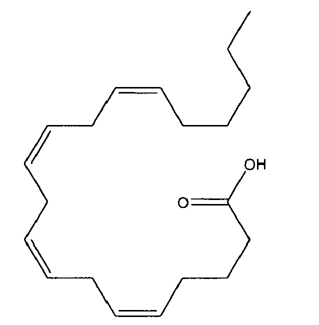

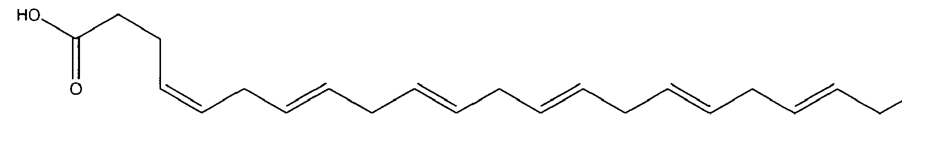

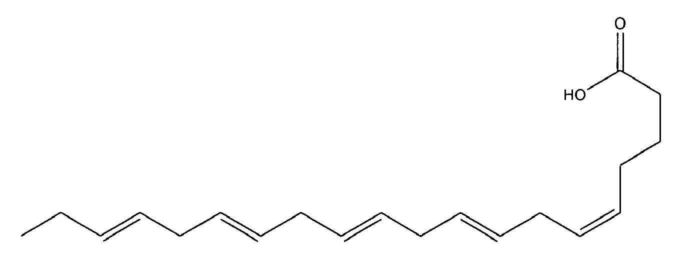

10. Use according to any one of claims 1 to 9, wherein the BACE mediated disease or condition involves overproduction of Amyloid β in neuronal or non neuronal cells of the brain.

11. Use according to claim 10, wherein the cells are neurons.

12. Use according to any one of claims 1 to 11, wherein the disease or condition involves the formation of Amyloid beta deposits in the brain.

13. Use according to any one of claims 1 to 12, wherein the disease or condition is a neurodegenerative disease.

14. Use according to any one of claims 1 to 13, wherein the disease or condition is Alzheimer's Disease.

15. Use according to any one of claims 1 to 13, wherein the disease or condition is selected from mild cognitive impairment, Down's syndrome, Hereditary Cerebral Hemmorhage with Amyloidosis of the Dutch-Type, cerebral Amyloid angiopathy, degenerative dementias, including dementias of mixed vascular and degenerative origin, dementia associated with Parkinson's disease, dementia associated with progressive supranuclear palsy, dementia associated with cortical basal degeneration and diffuse Lewy body type Alzheimer's disease.

16. A pharmaceutical composition comprising a therapeutically effective amount of one or more compounds of formula (I) as defined in any one of claims 1-9.

17. A pharmaceutical composition according to claim 16 for treating or preventing a disease or condition selected from Alzheimer's Disease, mild cognitive impairment, Down's syndrome, Hereditary Cerebral Hemmorhage with Amyloidosis of the Dutch-Type, cerebral Amyloid angiopathy, degenerative dementias, including dementias of mixed vascular and degenerative origin, dementia associated with Parkinson's disease, dementia associated with progressive supranuclear palsy, dementia associated with cortical basal degeneration and diffuse Lewy body type Alzheimer's disease.

18. A method for inhibiting BACE activity in a biological sample, comprising contacting said biological sample with an effective inhibitory amount of one or more compounds of formula (I) as defined in any of claims 1 to 9.

19. A method for treating or preventing a BACE mediated disease or condition comprising administering to a patient a therapeutically effective amount of one or more compounds of formula (I) as defined in any of claims 1 to 9.

20. A method according to any claim 19, wherein the disease or condition is selected from Alzheimer's Disease, mild cognitive impairment, Down's syndrome, Hereditary Cerebral Hemmorhage with Amyloidosis of the Dutch-Type, cerebral Amyloid angiopathy, degenerative dementias, including dementias of mixed vascular and degenerative origin, dementia associated with Parkinson's disease, dementia associated with progressive supranuclear palsy, dementia associated with cortical basal degeneration and diffuse Lewy body type Alzheimer's disease.
